(19) Europäisches Patentamt
European Patent Office
Office européen des brevets

(11) Publication number: 0 008 195 A1

# (12) EUROPEAN PATENT APPLICATION

(21) Application number: 79301541.3

(22) Date of filing: 01.08.79

(51) Int. Cl.[3]: A 61 L 15/07, A 61 L 15/00

(30) Priority: 04.08.78 US 930849

(43) Date of publication of application: 20.02.80 Bulletin 80/4

(84) Designated Contracting States: AT BE CH DE FR GB IT LU NL SE

(71) Applicant: Johnson & Johnson, 501 George Street, New Brunswick, New Jersey 08903 (US)

(72) Inventor: Evans, Anthony, 1735 Van Cortland Terrace, Teaneck, New Jersey (US)

(74) Representative: Grundy, Derek George Ritchie et al, CARPMAELS & RANSFORD 43, Bloomsbury Square, London WC1A 2RA (GB)

## (54) Orthopedic bandage.

(57) In an orthopedic bandage which hardens by means of a free radical catalyzed polymerization reaction, wherein said bandage comprises a cast forming composition including a solid water soluble vinyl monomer such as diacetoneacrylamide (DAA), N-isopropylacrylamide (N-IPA) or mixtures thereof, and at least one component of an improved redox catalyst system capable of initiating the polymerization of such vinyl monomer, said redox catalyst system comprising a copper containing compound in combination with a reducing agent, supported on a flexible carrier, the improvement which comprises increasing the effective working time of such bandage by providing in said bandage prior to or during polymerization thereof an alkaline earth metal salt, e.g. calcium sulfate. The copper containing compound may be cupric acetylacetonate.

EP 0 008 195 A1

# ORTHOPEDIC BANDAGE

## Field of the Invention

In an orthopedic bandage which hardens by means of a free radical catalyzed polymerization reaction, wherein said bandage comprises a cast forming composition including a solid, water soluble vinyl monomer such as diacetoneacrylamide (DAA), N-isopropylacrylamide (N-IPA) or mixtures thereof, and at least one component of an improved redox catalyst system capable of initiating the polymerization of such vinyl monomer, said redox catalyst system comprising a copper containing compound in combination with a reducing agent, supported on a flexible carrier, the improvement which comprises increasing the effective working time of such bandage by providing in said bandage prior to or during polymerization thereof an alkaline earth metal salt, e.g., calcium sulfate. The copper containing compound may be cupric acetylacetonate.

The polymerization of the above monomer may be initiated by contacting the bandage with water, in the presence of said redox catalyst system, e.g., by dipping the bandage, including the redox catalyst system supported thereon, in tap water.

The alkaline earth metal salt may be incorporated in the bandage and provides an orthopedic bandage having a constant working time despite the age or storage history of the bandage. Alternatively, the alkaline earth metal salt may be added to the dip water and will function to increase the working time of orthopedic bandages which have been subjected to conditions of heat and time sufficient to decrease the effective working time.

Background of the Prior Art

Plaster of Paris supported on fabric or gauze has been used almost exclusively in the preparation of surgical casts designed to immobilize and support portions of the body, e.g. a leg, arm, wrist, neck and the like. Plaster of Paris is inexpensive, convenient and ready to use after simply dipping in water. Moreover, practically all physicians, particularly orthopedic specialists, have long worked with the Plaster of Paris medium and are very familiar with the application. Once having mastered the art of working with plaster of Paris, they are reluctant to learn the different techniques associated with other media.

Notwithstanding, plaster of Paris has certain shortcomings. It is relatively heavy and can be damaged by wetting with water. It is also substantially opaque to x-rays, thus sometimes requiring that a cast be removed to ascertain, for example, whether a fracture has satisfactorily healed.

The various aforementioned problems with plaster of Paris orthopedic bandages have led to the development of orthopedic bandages such as the bandage described in U.S. Patent 3,630,194. This bandage utilizes as a cast forming composition a mixture including a water soluble monomer selected from the group consisting of DAA, N-IPA and mixtures thereof, said monomers being polymerizable, in the presence of water, by means of a redox catalyst system which comprises an oxidation component and a reducing agent. This bandage is hardened in a manner similar to the prior art plaster of Paris bandages by dipping the bandage into tap water. The advantage of this method of initiation is that, unlike certain other bandages which use thermoplastic sheets or apply hardenable resins from a paste, the technician working with the bandage does not have to learn new techniques for preparing a cast.

Although satisfactory in many ways, it has been found that the orthopedic bandages described in U.S. Pat. No.

3,630,194 suffered from certain drawbacks. For example, the rate of hardening was found to vary with the different tap waters used to initiate the polymerization reaction. Investigation of this phenomena surprisingly led to the discovery that the presence of different impurities in the tap water, such as copper, led to the non-uniform rates of hardening.

Furthermore, when both the oxidation and the reduction components of the catalyst were packages in the cast forming composition, as opposed to adding one or both of the components in the tap water, storage stability was found to be lacking.

These problems were solved by the orthopedic bandage disclosed in our Federal German Offenlegungschrift No. 2823340, by use of an improved redox catalyst system comprising a copper containing compound as the oxidising agent. However, bandages containing the copper containing compound, surprisingly, have been found to show a decrease in working time after storage at ambient conditions for one or more weeks. Thus, the orthopedist cannot be assured of a specific working time unless he knew the age and storage history of the particular bandage he was using. Of course, this made orthopedic bandages having the above improved redox catalyst system less desirable to the orthopedist.

Summary of the Invention

The instant invention relates to a novel orthopedic bandage, and a method for improving the storage stability and increasing the working time of orthopedic bandages which, comprise a cast forming composition comprising a redox catalyst system, including a copper containing compound in combination with a reducing agent, and a monomer, polymerizable by means of said redox catalyst system supported on a flexible carrier. The monomer is a water soluble, solid, vinyl monomer such as, for example DAA, N-IPA and mixtures thereof. The novelty of said bandage and the method for improving the storage stability and increasing the working time resides in incorporating an alkaline earth metal salt in such orthopedic bandages prior to polymerization thereof.

The orthopedic bandage so formulated is prepared for use by contacting it with an aqueous medium, preferably hot tap water, in the presence of a catalytic amount of the redox catalyst, including a copper containing compound whereby the vinyl monomer is polymerized. The polymerization catalyst may be added to the aqueous medium itself, or it may be incorporated into the cast forming composition. In the latter case, the bandage must be kept dry and out of contact with moisture laden air. Because both the copper salt and the reducing agent are required to initiate the polymerization reaction, one catalytic component may be excluded from the cast forming composition and added to the water at the time the bandage is dipped, thus minimizing the sensitivity of the bandage to water or moisture laden air.

It is preferred that both components of the catalyst are incorporated in the cast forming composition so that orthopedists need only dip the bandage in water in order to initiate polymerization and prepare the bandage for use. This simple procedure substantially duplicates, of course, the conventional techniques employed in preparing plaster of Paris casts. If the entire catalyst is not incorporated in the cast forming composition, the orthopedist will need to add any missing catalytic component to the water in which the bandage is immersed.

A bandage having both components of the catalyst incorporated in the cast forming monomer is known as a one package system and it is noted that the orthopedist, because of the convenience of use, prefers such a one package system. The one package embodiment of the instant bandage, that is a bandage including the alkaline earth metat salt as well as both components of the redox catalyst system as demonstrated below, has improved storage stability when compared to similar one package systems which do not include such alkaline earth metal salt.

Reducing agents, useful in preparing redox catalyst systems are known in the art and include ferrous sulfate, sodium sulfite, sodium dithionite, ferrous chloride, sodium formaldehyde sulfoxylate, oxalic acid, cobalt (II) chloride and hydrazine. All of the reducing agents known in the art as suitable components for redox catalyst systems, may be used in the practice of the instant invention.

The copper containing compound functions as the oxidizing agent of the redox catalyst system. Preferably, the copper containing compound is characterized by being partially soluble in water at room temperature. Very soluble copper salts (e.g., the sulfate and chloride) release such massive concentrations of copper ions in water that polymerization of the monomer is rapid and the setting time of a bandage is difficult to control. Partially soluble copper containing compounds (e.g., the acetylacetonate) release just enough copper ion to produce a bandage with a practical working time (60-70 seconds). Very insoluble copper salts (e.g., the phosphate) do not release enough copper ion to initiate polymerization and therefore would not be preferred if tap water was used to initiate the polymerization of the monomer.

Therefore, the most preferably copper containing compound is cupric acetylacetonate, and copper containing compounds including salts having similar solubilities in water at room temperature.

The copper containing compound may comprise as copper from .0005 to 5%, preferably from .01 to .10%, and most preferably from .01 to 0.10% by weight of the bandage (either incorporated in the bandage or in the dip water). The molar ratio of reducing agent to copper in said copper containing compound may vary from 100 to 1 to 5 to 1, preferably about 50 to 1.

It is believed that the copper containing compound, especially cupric acetylacetonate, undergoes auto oxidation when the above-described bandages are aged, thereby generating a more reactive oxidizing specie. Thus, the aged bandages are found to have a decreased working time due to the accelerating of the polymerization rate of the monomer upon dipping the bandage in water. Of course, to solve this problem, the copper containing compound can be added to the dip water just prior to the initiation of polymerization for convenience; however, as discussed above, it is desirable to incorporate both components of the redox catalyst system in the bandage.

It has now been unexpectedly found that providing an alkaline earth metal salt in the bandage will act to moderate the polymerization rate of the aged bandage thus providing a suitable working time. The alkaline earth metal salt can be initially formulated into the cast forming composition, impregnated into the aged bandage prior to dipping in tap water for the purpose of initiating polymerization, or incorporated into the dip water. In each case, the alkaline earth metal salt acts to moderate the rate of polymerization and thereby provide a suitable working time. For example, as shown below, a bandage having a working time of about 40 seconds after aging for 3 weeks at 38°C (100°F) had a working time of 111 seconds when formulated with 600 weight percent, based on copper of $CaSO_4$.

While not wishing to be bound by theory, it is believed that on aging without $CaSO_4$, or other alkaline earth metal salt, cupric acetylacetonate and similar copper catalysts undergo autooxidation to yield a more reactive specie which is believed to be more water soluble. $CaSO_4$ or other alkaline earth metal salt may be added to the chemical formulation, then when the bandage was dipped and as it was being applied, the copper, be it in the +1 or +2 state, would be rapidly converted back to copper acetylacetonate (or other similar copper catalyst) to yield a more controllable bandage as far as rate of polymerization is concerned.

The alkaline earth metal salt may be selected from the group consisting of magnesium, calcium, strontium and and barium salts. The anions of said salts will be selected so that the salt will at least slightly soluble in the dip water. The common salts are effective for the purposes of this invention and may be chosen on the basis of their cost and innocuous nature. For example, $CaCl_2$, $MgSO_4$, $SrNO_3$, $BaCO_3$, etc. may be used. The most preferred alkaline earth metal salt is $CaSO_4$. It appears to be important that the salt be an alkaline earth metal salt since potassium sulfate, ammonium sulfate, and sodium sulfate were found to be ineffective for the purposes of the instant invention.

The alkaline earth metal salt can be impregnated on the bandage by means of the techniques disclosed in U.S. Patent No. 3,630,194, during the initial formulation or can be applied to an aged bandage from a slurry in a non-reactive solvent such

as methylene chloride, methanol, 2-butanone or applied to the bandage as a finely divided powder.

In order to insure the moderation the effective working time of the bandage, an amount of at least 300 weight percent based on copper is preferred, more preferably, from 300 to 900%. These same amounts may be used when the alkaline earth metal salt is incorporated in the dip water.

It is preferred that the alkaline earth metal salt be incorporated during the initial formulation of the bandage thereby assuring an extended shelf like. It has also been found that the ratio of redox catalyst to monomer is not as critical when the alkaline earth metal salt is incorporated in the initial formulation. Finally, the bandages including the alkaline earth metal salt are found to be more creamy during application thereby increasing conformability and easing application.

The cast forming composition preferably comprises from 50 to 100% more preferably 65 to 75%, by weight based on the weight of the flexible carrier. Of the total solids in the cast forming composition, the monomer preferably comprises from about 75 to 100% more preferably 85 to 95% by weight of the total. The remainder will include binders, fillers, co-monomers (other than the water soluble, solid, vinyl monomers), the redox catalyst components (if incorporated in the cast forming composition), etc.

The instant novel bandages may be prepared, packaged and used in a manner similar to the bandage described in U.S. Patent No. 3,630,194. Materials suitable for preparing said novel bandage (other than the redox catalyst system and the monomers, but including the flexible carrier, co-monomers, binders, fillers, polymerization rate controllers, etc.) are also disclosed in said patent and the disclosure of said patent is hereby incorporated by reference to describe such materials as well as the methods of preparation, packaging and use of the instant novel bandages. No buffers are necessary for the action of copper in the orthopedic bandage of the instant invention. When persulfates are used as initiators, large amounts of persulfates are required. Since persulfates decompose to acidic by-products which are harmful to skin, a buffer is necessary. Because so little copper may be used in the instant novel bandages, the

pH of the wet bandage does not change. Sodium sulfite, the preferred co-reactant with cupric acetylacetonate, acts as its own buffer.

The preferred flexible carrier for preparing the instant novel bandages is fiberglass. Fiberglass is preferred since it adds to the strength of the cast, especially during the earlier stages following activation that is, fiberglass contributes to the "green strength" of casts formed from the bandage. Fiberglass is a very brittle fiber and fabrics prepared therefrom are especially notable for their non-conformability. The incorporation of a poly glycol, preferably a 1,2 ditertiary glycol, in a bandage utilizing a fiberglass fabric provides conformability and ease of wrapping without detracting from the desirable strength properties of fiberglass based bandages.

Suitable 1,2 ditertiary glycols which may be used are described by the general formula:

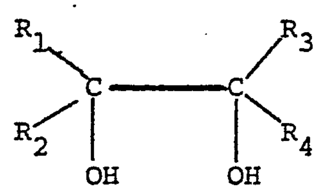

where $R_1$, $R_2$, $R_3$ and $R_4$ are alkyl groups, e.g., $C_1$ to $C_5$ alkyl groups.

The most preferred 1,2 ditertiary glycol is pinacol (where $R_1 = R_2 = R_3 = R_4 = CH_3$). The 1,2 ditertiary glycol should be incorporated at a level of from 0.2 to 10% preferably 0.5 to 2% of the total weight of the coated bandage to improve the conformability of said bandage.

The following examples illustrate the above described invention, however, there is no intent to limit the claims thereto.

EXAMPLE 1: Comparison of Working Times of a Bandage with and without $CaSO_4$ added to Dip Water

A polymerization mixture comprising:

| | |
|---|---|
| 975 grams | Diacetone Acrylamide |
| 20 grams | Carbowax 4000 (polyethylene oxide available from Union Carbide Corp., New York) |
| 30 grams | Silane treated Fumed Silica |
| 43 grams | Pinacol |
| 10 grams | 3A Molecular Sieve (available from Linde Div., of Union Carbide Corp, New York) |
| 3.0 grams | Mixture of Diacetoneacrylamide and t-butylcatechol (weight ratio $3.33 \times 10^{-3}$ t-butylcatechol to 1 DAA) |

was coated on a 4inch (100mm) strip of fiberglass. The strip was run through a bath comprising said mixture at a speed of 30 feet/minute (9 metres/minute). The weight percent pickup was $37 \pm 1.7$. A catalyst mixture comprising 106 grams of a 24/1 (by weight) preblend of sodium sulfite and cuprous acetylacetonate, 694 grams of sodium sulfite, 22 grams of a high molecular weight polyethyleneoxide such as Polyox Coagulant Grade (Union Carbide Corp., New York), was dusted onto the coated strip at a rate of about 15.5 grams per minute to yield a catalyst to diacetoneacrylamide ratio of from .085 to .094. A sample of said coated strip including catalyst, when tested as an orthopedic bandage by the procedure described in the working examples of the abovementioned Offenlegungschrift 2823340 had a working time of 64-68 secs. The remainder of the coated strip was held for approximately two months at room temperature and used for the comparison below made.

Sample A and B are the controls (i.e., after two months ageing at room temperature) and in Samples C, D and E, 1 gram of $CaSO_4$ was added to 1 litre of dip water prior to activating the bandage. A three-yard (2.75 metre) strip is used in this test.

The working time of a bandage is the time, in seconds, from the immersion of a bandage in water to the time that the wrapper feels that he can not unroll the bandage any further without exerting pressure. If the wrapper does not have to exert pressure at any time, the working time is simply the time elapsed in unrolling the bandage completely. When the wrapper does exert pressure, the polymerizing monomer has become too tacky to allow the bandage to be unrolled easily.

Note that the instant novel bandages (Samples C, D and E) show improved working time. The working time of the bandage which was "aged" two months at room temperature and treated by the process of the instant invention is equal to or greater than the working time obtained when the bandage was fresh.

| | A | B | C | D | E |
|---|---|---|---|---|---|
| Working Time (seconds) | 44 | 52 | 67 | 78 | 76 |

EXAMPLE 2: Comparison of Working Times of a Bandage with and Without $CaSO_4$ (solid) added directly to Bandage Formulation

A bandage was prepared in a manner similar to the bandage of Example 1, except that the weight ratio of catalyst to diacetone acrylamide was held between .08 and .065. The initial working time was 53 seconds but after 2 weeks at 38°C (100°F), the working time had decreased to 40 seconds. It was found that increasing amounts of $CaSO_4$ acted to progressively extend the working time of "aged" bandages. To prepare the samples for testing, various amounts of $CaSO_4$ were placed in foil lined storage bags along with the bandage. The bags were sealed and shaken to disperse the $CaSO_4$ into the bandages. Below are the working times (seconds) obtained.

| NO. OF WEEKS AT 100°F (38°C) | GRAMS OF $CaSO_4$ ADDED TO STORAGE BAG | | | |
|---|---|---|---|---|
| | 0.0 | 0.5 | 1.0 | 2.0 |
| 0 | 53 | - | | |
| 1 | 45 | 70 | 108 | 105 |
| 2 | 40 | 79 | 100 | 111 |

CLAIMS:

1. A method for increasing the effective working time of an orthopedic bandage which hardens by means of a free radical catalyzed polymerization reaction, wherein said bandage comprises a cast forming composition including a solid, water soluble vinyl monomer and at least one component of a redox catalyst system capable of initiating the polymerization of such vinyl monomer, said redox catalyst system comprising a copper containing compound in combination with a reducing agent, supported on a flexible carrier, characterized by providing in said bandage prior to or during polymerization thereof an alkaline earth metal salt.

2. The method of Claim 1 wherein said alkaline earth metal salt is selected from the group consisting of the sulfates of calcium, barium, strontium and magnesium.

3. The method of Claim 1 wherein said alkaline earth metal salt is provided in an amount of at least 300 weight per cent based on the weight of the copper.

4. The method of Claim 1 wherein said copper containing compound comprises, as copper, from .0005 to 5 per cent by weight of said bandage.

5. The method of Claim 1 wherein said vinyl monomer is selected from the group consisting of diacetoneacrylamide (DAA), N-isopropylacrylamide (N-IPA) or mixtures thereof.

6. An orthopedic bandage which hardens by means of a free radical catalyzed polymerization reaction, and which has improved storage stability and increased working time, wherein said bandage comprises a cast forming composition supported on a flexible carrier, said cast forming composition including a solid, water soluble vinyl monomer, and a redox catalyst system comprising a copper containing compound in combination with a reducing agent, and being characterized by containing an alkaline earth metal salt.

7. The bandage of Claim 6 wherein said alkaline earth metal salt is selected from the group consisting of the sulfates of calcium, barium, strontium and magnesium.

8. The bandage of Claim 6 wherein said alkaline earth metal salt is provided in said bandage in an amount of at least 300 weight per cent based on the weight of the copper.

9. The bandage of Claim 6 wherein said copper containing compound comprises, as copper, from .0005 to 5 per cent by weight of said bandage.

10. The bandage of Claim 6 wherein said vinyl monomer is selected from the group consisting of diacetoneacrylamide (DAA), N-isopropylacrylamide (N-IPA) or mixtures thereof.

European Patent Office

# EUROPEAN SEARCH REPORT

Application number

EP 79 301 541.3

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| X,P | FR - A1 - 2 392 678 (JOHNSON & JOHNSON) * claims 1 to 4 * -- | 1,3, 5,6, 8,10 | A 61 L 15/07 A 61 L 15/00 |
| X | FR - A1 - 2 239 256 (KENDALL) * claims 1 and 2 * -- | 1,2,6, 7 | |
| A | DE - A1 - 2 758 216 (UNION CARBIDE) * complete document * -- | | |
| A | US - A - 4 078 568 (NORTHERN ILLINOIS RESEARCH) * complete document * ---- | | |

TECHNICAL FIELDS SEARCHED (Int.Cl.3)

A 61 L 15/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 29-10-1979 | SCHULTZE |